# EUROPEAN PATENT APPLICATION

(11) **EP 1 920 713 A2**
(43) Date of publication of application: **14.05.2008**
(21) Application number: 07021744.3
(22) Date of filing: 07.11.2007
(51) Int. Cl.: A61B 5/103

(54) **Goniometer for measuring artificial acetabular cup angles and method for measuring thereof using the goniometer**

(30) Priority: 13.11.2006 KR 20060111638
(71) Applicant: Korea Advanced Institute of Science and Technology, Yuseong-Gu, Daejeon 305-701 (KR)
(72) Inventor: Yoon, Yong San, Daejeon 305-707 (KR)
(74) Representative: Gassner, Wolfgang

(57) **Abstract**

A goniometer for measuring artificial acetabular cup angles having the function of measuring an inclination angle and anteversion angle and a method for measuring the artificial acetabular cup angle using the same are disclosed. This goniometer measures the inclination angle or anteversion angle by using the first angle measuring unit, and then measures the inclination angle or anteversion angle by using the second angle measuring unit. Accordingly, it becomes possible to obtain precise operation angles of the acetabular cup easily and quickly, so it is possible to perform the artificial hip joint operation more precisely and quickly.

## Description

### BACKGROUND OF THE INVENTION

This application claims priority to Korean Patent Application No. 2006-0111638, filed on November 13, 2006, in the Korean Intellectual Property Office, the entire contents of which are hereby incorporated by reference.

### 1. Field of the Invention

The present invention relates to a goniometer for measuring artificial acetabular cup angles having the function of measuring the operative inclination angle and the operative anteversion angle and a method for measuring thereof using the same, more specifically to a goniometer for measuring artificial acetabular cup angles and a method for measuring the artificial cetabular cup angles using the same by which it is possible to insert the acetabular cup in the pelvis in an accurate direction by measuring the operative inclination angle and the operative anteversion angle during artificial acetabular cup operation.

### 2. Description of the Related Art

In general, a hip joint refers to a joint composed of the femoral capital having a spherical shape and the pelvis acetabulum of a socket shape.

If such a hip joint is damaged for various reasons, operation is performed to remove the joint portion and replace it with an artificial hip joint. Such an operation is called an artificial hip joint operation or hip joint replacement operation.

An artificial hip joint consists of a portion that is inserted into the femur to replace the femoral capital and a portion that is inserted into the pelvis to replace the acetabulum. In such an artificial hip joint operation, an acetabular cup that is to replace the acetabulum of pelvis should be inserted into the pelvis in an accurate direction.

If the acetabular cup is not accurately inserted into the correct position in the pelvis during artificial hip joint operation, the life of the artificial acetabular cup becomes short, and there could be a problem of dislocation in a serious case.

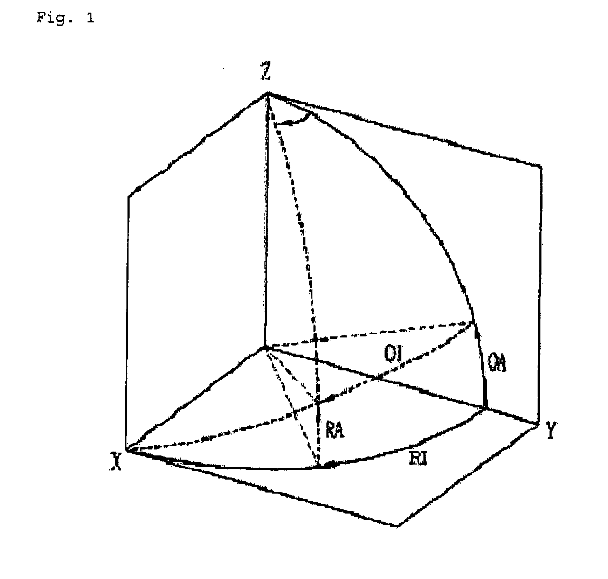

Fig. 1 is a schematic view for describing an operative anteversion angle, operative inclination angle, radiographic anteversion angle, and radiographic inclination angle for artificial hip joint operation.

As shown in Fig. 1, the acetabular cup angle can be shown by the operative inclination angle OI and the operative anteversion angle OA or the radiographic inclination angle RI and radiographic anteversion angle RA. The operative inclination angle OI and the operative anteversion angle OA are angles that doctors use mainly when performing an operation on the acetabular cup, and the radiographic inclination angle RI and radiographic anteversion angle RA are angles used mainly during X-ray photographing. In an artificial hip joint operation, the operative inclination angle OI and the operative anteversion angle OA, or the radiographic inclination angle RI and radiographic anteversion angle RA are measured, and an acetabular cup shaft (inserter) is advanced according to the measured angles to accurately insert the acetabular cup at the correction position in the pelvis.

Meanwhile, the approximate life of the acetabular cup is generally about 10 years, but as the average human life span is lengthened, there are cases in which it is necessary to replace the acetabular cup that has expired with a new one. In such a case, a new acetabular cup cannot be inserted at an accurate angle unless the angle of the acetabular cup that was inserted in the previous operation is measured.

For example, U.S. Patent No. 6,743,235 discloses an acetabular cup navigation apparatus for inserting the acetabular cup into the proper position by navigating according to a given angle. This apparatus is provided with a horizontal measuring instrument and a laser pointer as a means for guiding the acetabular cup to a given angle. However, the apparatus disclosed in this patent can be used only in the artificial hip operation, and cannot provide a function of measuring the angle of the acetabular cup that was already operated on.

In addition, U.S. Patent No. 6,214,014 discloses an alignment apparatus for artificial hip joint operation. But the apparatus disclosed in this patent can measure only the operative inclination angle, so it is difficult to insert the acetabular cup in an accurate direction during artificial hip joint operation.

### SUMMARY OF THE INVENTION

The present invention is to solve the aforementioned problems with an object to provide a goniometer for measuring artificial acetabular cup angles and a method for measuring the artificial acetabular cup angles using the same by which it is possible to easily and quickly obtain a precise operative angle of the acetabular cup by measuring the operative inclination angle using a first angle measuring unit and the operative anteversion angle using a second angle measuring unit.

In accordance with one aspect of the present invention, there is provided a goniometer for measuring artificial acetabular cup angles having a function of measuring an inclination angle and anteversion angle of the artificial acetabular cup inserted into the pelvis of the person operated on, the goniometer comprising: a body block; a direction indicating member which is fixed on one side of the body block and is provided with a plurality of direction indicating rods; a first angle measuring unit which is rotatably mounted on the body block and includes a first indicator and a first rotating graduation plate; a second angle measuring unit which is capable of rotating around a second rotating shaft that is not in parallel with a first rotating shaft of the first angle measuring unit and includes a second indicator and a second rotating graduation plate for measuring the rotating angle thereof; a connecting rod which is connected to the second rotating graduation plate of the second angle measuring unit so as to mount the acetabular cup; and a detachable member installed at one end of the connecting rod for mounting the acetabular cup shaft selectively.

In accordance with another aspect of the present invention, there is provided a method for measuring artificial acetabular cup angles of the person operated on by using the goniometer of Claim 1 for measuring the artificial acetabular cup angle having the function of measuring an inclination angle and anteversion angle, the method comprising the steps of: fixing a reference member for marking the pelvis plane of the person operated on to the pelvis of the person operated on; inserting the acetabular cup shaft into the acetabular cup that is operated on of the pelvis of the person operated on; and measuring the inclination angle or the anteversion angle by rotating the first rotating graduation plate of the first angle measuring unit in such a way that the coordinates indicated by a plurality of direction indicating rods of the direction indication member and the coordinates indicated by the reference member of the pelvis of the patient operated on agree, and measuring the inclination angle or the anteversion angle by rotating the second rotating graduation plate of the second angle measuring unit.

Features of the present invention described above and other advantages will be more clearly understood by the following non-limited examples, which are not intended to restrict the scope of the invention but are instead illustrative embodiments of the present invention.

### BRIEF DESCRIPTION OF THE DRAWINGS

These and other objects, features, aspects, and advantages of the present invention will be more fully described in the following detailed description for preferred embodiments and examples, taken in conjunction with the accompanying drawings. In the drawings:
Fig. 1 is a schematic view for describing an operative anteversion angle, operative inclination angle, radiographic anteversion angle, and radiographic inclination angle for artificial hip joint operation;
Fig. 2 is an exploded perspective view showing a goniometer for measuring the artificial acetabular cup angle having the function of measuring the operative inclination angle and operative anteversion angle according to a preferred embodiment of the present invention;
Fig. 3 is a perspective view showing the assembled state of the goniometer for measuring the artificial acetabular cup angle having the function of measuring the operative inclination angle and operative anteversion angle according to the preferred embodiment of the present invention; and
Fig. 4 is a plan view showing a goniometer for measuring the artificial acetabular cup angle having the function of measuring the operative inclination angle and operative anteversion angle according to the preferred embodiment of the present invention.

### DETAILED DESCRIPTION OF THE INVENTION

Below will be described in detail a goniometer for measuring the artificial acetabular cup angle and an artificial acetabular cup angle measuring method using the same with reference to the accompanying drawings.

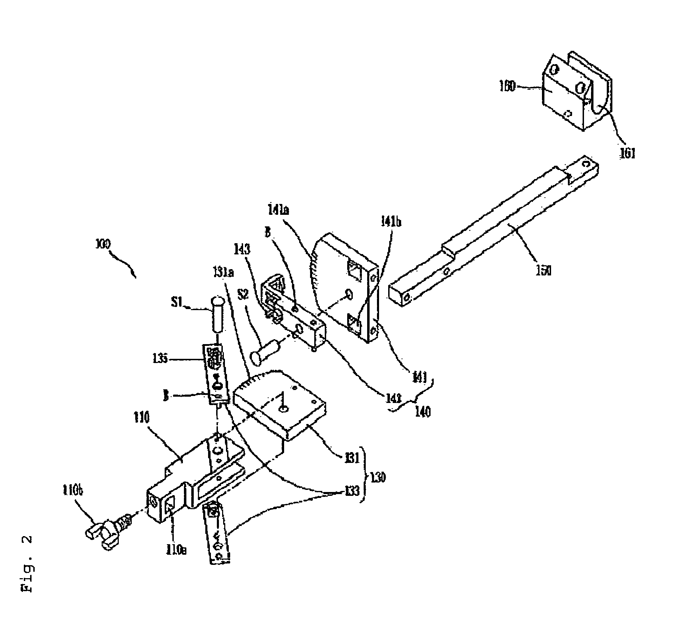

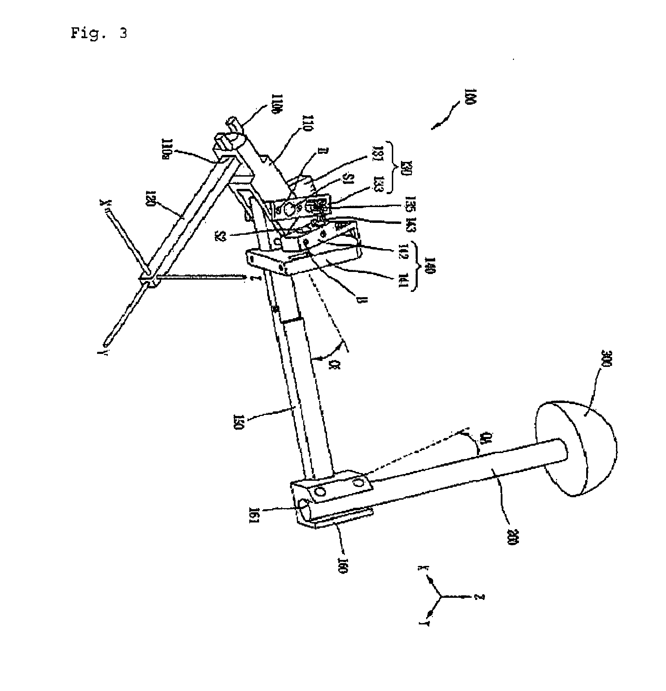

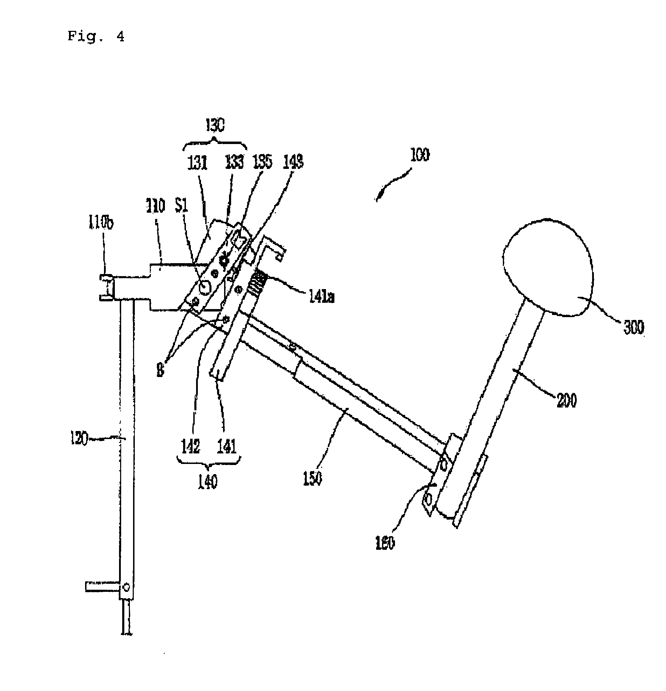

Fig. 2 is an exploded perspective view showing a goniometer for measuring the artificial acetabular cup angle having the function of measuring the operative inclination angle and operative anteversion angle according to the preferred embodiment of the present invention, and Fig. 3 is a perspective view showing the assembled state of the goniometer for measuring the artificial acetabular cup angle illustrated in Fig. 2, and Fig. 4 is a plan view of the goniometer for measuring the artificial acetabular cup angle illustrated in Fig. 2.

As shown in these drawings, a goniometer 100 for measuring the artificial acetabular cup angle according to the preferred embodiment of the present invention generally comprises a body block 110, a direction indicating member 120, a first angle measuring unit 130, a second angle measuring unit 140, a connecting rod 150, and a detachable holder 160 as a detachable holder.

Here, the body block 110 has a shape with one end branched into two. The direction indicating member 120 is fixed on one side of the body block 110, and has three direction indicating rods extending to X, Y and Z axes respectively.

The first angle measuring unit 130 is rotatably mounted on the center of both branched portions of the body block 110, and is provided with a first rotating graduation plate 131 which has a plurality of graduations marked on the outer surface, and first indicators 133 which are fixed to the body block 110 so as to confirm the angle of rotation by indicating any one graduation of the graduations 131a marked on the first rotating graduation plate 131.

The second angle measuring unit 140 is provided with a second rotating graduation plate 141 which is rotated in the direction crossing at right angles the rotation direction of the first rotating graduation plate 131 and has a plurality of graduations 141a marked on the outer surface thereof, and a second indicator 142 which is fixed on one side of the first rotating graduation plate 131 so as to confirm the angle of rotation by indicating any one graduation of the graduations 141a marked on the second rotating graduation plate 141. The second rotating graduation plate 141 is rotatably mounted on the second indicator 142.

The second rotating graduation plate 141 is provided with assembly slots 141b formed therein and one end of the connecting rod 150 is fixed to the second rotating graduation plate 141 through assembly slots 141b. And at the other end of the connecting rod 150 is installed the detachable holder 160 for an acetabular cup shaft 200 to be mounted selectively.

In more detail, the first rotating graduation plate 131 is installed rotatably in the branched portion of the body block 110 by a first rotating shaft S1. The first indicators 133 are fixed to the top surface and bottom surface of the body block 110 by bolts B. To the first rotating graduation plate 131 is fixed the second indicator 142 by bolts B, and on the second indicator 142 is installed rotatably the second rotating graduation plate 141 by a second rotating shaft S2. Namely, the second rotating graduation plate 141 is installed rotatably around the second rotating shaft S2 while arranged in the direction crossing at right angles to the first rotating graduation plate 131. At this time, the first rotating shaft S1 and the second rotating shaft S2 are arranged in a direction mutually crossing at right angles.

One end of the direction indicating member 120 is inserted into an assembly slot 110a formed at one end of the body block 110 and fixed thereto by a fastening bolt 110b. At the other end of the direction indicating member 120 are fixed three direction indicating rods extended to X, Y and Z axes respectively.

One end of the connecting rod 150 is inserted in the assembly slot 141b formed in the second rotating graduation plate 141 and fixed thereto by bolt. The other end of the connecting rod 150 is combined with the detachable holder 160.

The detachable holder 160 is fixed to the other end of the connecting rod 150 by a fastening element such as a bolt so that the acetabular cup shaft 200 can be selectively mounted or separated. In the detachable holder 160 is formed a slot 161 in which the acetabular cup shaft 200 is mounted.

And, a first clamping bolt 135 is installed on the body block 110 for fixing the first rotating graduation plate 131, a second clamping bolt 143 is installed on the second indicator 142 for fixing the second rotating graduation plate 141. Here, the first clamping bolt 135 plays a role of fixing the first rotating graduation plate 131 at a desired angle after the operator rotates the first rotating graduation plate 131 to a desired angle, and the second clamping bolt 143 plays a role of fixing the second rotating graduation plate 141 at a desired angle after the operator rotates the second rotating graduation plate 141 to a desired angle.

In the operation of artificial hip joint to replace the previously operated acetabular cup (which is to be replaced) with a new acetabular cup, it is possible to easily measure the operation angle of the new acetabular cup while easily separating the old acetabular cup using the artificial acetabular cup angle measuring goniometer according to the preferred embodiment of the present invention.

The method for measuring the angle of the acetabular cup using the goniometer 100 for measuring the artificial acetabular cup angle of the present invention is described below.

First, a reference member for marking the pelvis plane of the person operated on is fixed to the pelvis of the person operated on. At this time, to measure the pelvis plane of the person operated on, an "acetabular cup navigation apparatus" disclosed in Korean Patent Application No. 10-2005-0030405 can be used.

When the reference member is fixed on the pelvis of the person operated on, the acetabular cup shaft 200 is inserted into the acetabular cup to be replaced, and then the direction of the acetabular cup 200 is fixed.

Next, the first rotating graduation plate 131 of the first angle measuring unit 130 is rotated so that the angle direction indicating rod of the direction indicating member 120 agrees with the direction indicated by the reference member to measure the operative inclination angle OI, and the second rotating graduation plate 141 of the second angle measuring unit 140 is rotated to measure the operative anteversion angle OA.

In addition, the goniometer 100 for measuring the artificial acetabular cup angle according to the embodiment of the present invention can be used also to measure the radiographic inclination angle RI and the radiographic anteversion angle RA.

Above was described the method of using the goniometer 100 for measuring the artificial acetabular cup angle according to the embodiment of the present invention to measure the inclination angle and anteversion angle, but the goniometer 100 for measuring the artificial acetabular cup angle can be used also for the operation of an artificial hip joint.

First, use the first angle measuring unit 130 to adjust the acetabular cup 300 to match the operative inclination angle OI to be operated at. That is, adjust the operative inclination angle OI by rotating the first rotating graduation plate 131 around the first rotating shaft S1 so that the graduation 131a of the first rotating graduation plate 131 and the first indicator 133 become 20 degrees, for example, for the operative inclination angle OI. In this condition, tighten the first clamping bolt 135 to fix the angle of the first rotating graduation plate 131.

Then, use the second angle measuring unit 140 to adjust the acetabular cup 300 to match the operative anteversion angle OA to be operated at. That is, adjust the operative anteversion angle OA by rotating the second rotating graduation plate 141 around the second rotating shaft S2 so that the graduation 141a of the second rotating graduation plate 141 and the second indicator 142 become 40 degrees, for example, for the operative anteversion angle OA. In this condition, tighten the second clamping bolt 143 to fix the angle of the second rotating graduation plate 141.

After adjusting the operative inclination angle and the operative anteversion angle like above, match the angle direction indicating rod of the direction indicating member 120 to the reference member fixed on the pelvis of the person operated on. In this condition, while advancing the acetabular cup shaft 200 toward the pelvis of the patient, insert the acetabular cup 300 into the pelvis to complete the operation of artificial hip joint.

According to the present invention, it is possible to obtain the precise operation angle of the acetabular cup easily and quickly by measuring the operative inclination angle using the first angle measuring unit and then measuring the operative anteversion angle using the second angle measuring unit, and perform the artificial hip joint operation more precisely and quickly.

while the present invention has been described with reference to the preferred embodiments, these are intended to illustrate the invention and do not limit the scope of the present invention. It will be obvious to those skilled in the art that various modifications and/or variations may be made therein without departing from the spirit and scope of the present invention.

## Claims

1. A goniometer for measuring artificial acetabular cup angles having a function of measuring an inclination angle and anteversion angle of the artificial acetabular cup inserted into the pelvis of the person operated on, the goniometer comprising:
a body block;
a direction indicating member which is fixed on one side of said body block and is provided with a plurality of direction indicating rods;
a first angle measuring unit which is rotatably mounted on said body block and includes a first indicator and a first rotating graduation plate;
a second angle measuring unit which is capable of rotating around a second rotating shaft that is not in parallel with a first rotating shaft of said first angle measuring unit and includes a second indicator and a second rotating graduation plate for measuring the rotating angle thereof;
a connecting rod which is connected to said second rotating graduation plate of said second angle measuring unit so as to mount the acetabular cup; and
a detachable member installed at one end of said connecting rod for mounting the acetabular cup shaft selectively.

2. The goniometer of Claim 1, wherein the first rotating graduation plate of said first angle measuring unit is rotatably mounted on said body block and said first indicator is fixed on said body block.

3. The goniometer of Claim 2, wherein the first rotating shaft of said first rotating graduation plate is in parallel with any one direction indicating rod of the plurality of direction indicating rods of said direction indication member.

4. The goniometer of Claim 3, wherein one end of said direction indicating member is inserted into an assembly slot formed on said body block and fixed thereto by a fastening bolt.

5. The goniometer of Claim 1, wherein said second angle measuring unit is mounted at right angles with the first rotating shaft of said first angle measuring unit.

6. The goniometer of Claim 5, wherein the second rotating graduation plate is rotated around the second rotating shaft crossing at right angles with the first rotating shaft of said first indicator, and said second indicator is fixed on said first rotating graduation plate.

7. The goniometer of Claim 6, wherein one end of said connecting rod is inserted in an assembly slot formed on said rotating graduation plate and fixed thereto by a bolt.

8. The goniometer of Claim 1, wherein a first clamping bolt for fixing the angle of rotation is installed on said first rotating graduation plate located on one side of said body block, and a second clamping bolt for fixing the angle of rotation is installed on said second rotating graduation plate.

9. The goniometer of Claim 1, wherein said direction indication member is provided with three (X, Y and Z axes) direction indicating rods, and respective direction indicating rods are arranged in the directions mutually crossing at right angles.

10. The goniometer of Claim 1, wherein said connecting rod is arranged in parallel with the second rotating shaft of said second angle measuring unit.

11. A method for measuring artificial acetabular cup angles of the person operated on by using the goniometer of Claim 1 for measuring the artificial acetabular cup angle having the function of measuring an inclination angle and anteversion angle, the method comprising the steps of:
fixing a reference member for marking the pelvis plane of the person operated on to the pelvis of the person operated on;
inserting the acetabular cup shaft into the acetabular cup that is operated on of the pelvis of the person operated on; and
measuring the inclination angle or the anteversion angle by rotating the first rotating graduation plate of the first angle measuring unit in such a way that the coordinates indicated by a plurality of direction indicating rods of the direction indication member and the coordinates indicated by the reference member of the pelvis of the patient operated on agree, and measuring the inclination angle or the anteversion angle by rotating the second rotating graduation plate of said second angle measuring unit.
